# EUROPEAN PATENT SPECIFICATION

(11) **EP 3 934 532 B1**
(45) Date of publication and mention of the grant of the patent: **28.01.2026**
(21) Application number: 20714808.1
(22) Date of filing: 04.03.2020
(51) Int. Cl.: A61B 5/11, G16H 50/30, G16H 50/20, G16H 50/50, G16H 20/40, G16H 20/30, A61B 5/103, G16H 50/70, A61B 17/00

(54) **QUANTITATION OF SECONDARY DEGRADATION IN UNAFFECTED JOINTS RESULTING FROM OSTEOARTHRITIS OF THE KNEE OR HIP**
QUANTIFIZIERUNG DER SEKUNDÄREN DEGRADATION IN NICHT BETROFFENEN GELENKEN ALS FOLGE VON ARTHROSE DES KNIES ODER DER HÜFTE
QUANTIFICATION DE LA DÉGRADATION SECONDAIRE DANS LES ARTICULATIONS NON AFFECTÉES RÉSULTANT DE L'ARTHROSE DU GENOU OU DE LA HANCHE

(30) Priority: 04.03.2019 US 201962813491 P
(43) Date of publication of application: 12.01.2022
(73) Proprietor: Smith&Nephew, Inc., Memphis, Tennessee 38116 (US); Smith & Nephew Asia Pacific Pte. Limited, Singapore 138565 (SG); Smith & Nephew Orthopaedics AG, 6300 Zug (CH)
(72) Inventor: MCKINNON, Brian, Cordova, Tennessee 38016 (US)
(74) Representative: Appleyard Lees IP LLP
(86) International application number: PCT/US2020/020866
(87) International publication number: WO 2020/180916

(56) References cited:
- WO-A1-2017/214656
- US-A1- 2011 112 808
- US-A1- 2013 185 310
- CLARE K. FITZPATRICK ET AL: "Comparison of patellar bone strain in the natural and implanted knee during simulated deep flexion", JOURNAL OF ORTHOPAEDIC RESEARCH, vol. 29, no. 2, 9 September 2010 (2010-09-09), US, pages 232 - 239, XP055690361, ISSN: 0736-0266, DOI: 10.1002/jor.21211

## Description

This patent application claims priority to U.S. Provisional Patent Application No. 62/813,491, titled "QUANTITATION OF SECONDARY DEGRADATION IN UNAFFECTED JOINTS RESULTING FROM OSTEOARTHRITIS OF THE KNEE OR HIP," filed on March 4, 2019.

### BACKGROUND

Osteoarthritis is one of the most common forms of arthritis, affecting millions of people worldwide. It generally occurs when protective joint cartilage that cushions the ends of bones gradually deteriorates or wears down over time. Healthy cartilage is a firm, yet slippery tissue that permits nearly frictionless joint motion. However, when osteoarthritis occurs, the slick surface of the cartilage becomes rough, which negatively impacts joint motion. If the cartilage wears down completely, the individual may be left with one bone rubbing directly on another bone. Without diagnosis and treatment, osteoarthritis can lead to serious complications that can affect an individual's mobility.

Osteoarthritis is most commonly reported in knee joints and is primarily observed in the medial compartment of the joint, which is located near the middle of the knee on the inner side. This is primarily due to the fact that the loads transferred through the medial compartment during many activities (including walking) are substantially higher than in the lateral compartment. The characterization of joint loading and the associated biomechanics in the presence of osteoarthritis have been analyzed and studied extensively. Current corrective procedures are mainly directed toward total or partial joint replacement. The goal of the corrective procedure is to restore joint mechanics and loading schemes to their original function by replacing the affected tissue with metallic and polymeric components.

Deciding when to undergo surgery to treat osteoarthritis ultimately lies with the patient, but often a patient's clinician may suggest that the surgery be deferred as long as possible. This is because delaying surgery reduces the applied service life of joint replacement implants, and thus decreases the likelihood of implant failure and costly revision surgery. However, the decision to delay surgery can be harmful to the patient's unaffected and otherwise healthy joints.

It has been demonstrated that compensatory gait changes associated with knee osteoarthritis can increase the magnitude and loading rate in joints adjacent to the affected knee. Increased loading rate can drive the progression of existing osteoarthritis and the onset of osteoarthritis in these adjacent joints. *See* Mündermann, Annegret, Chris O. Dyrby, and Thomas P. Andriacchi, Secondary gait changes in patients with medial compartment knee osteoarthritis: increased load at the ankle, knee, and hip during walking," Arthritis & Rheumatology 52.9 (2005): 2835-2844. These secondary effects of loading on the patient's contralateral knee, hips, ankles, and back may be irreversible. Therefore, when making the decision to undergo surgery it is of interest to determine the extent to which the knee with osteoarthritis is affecting a patient's other joints. For example, a clinician may advise a patient with mild osteoarthritis in the medial compartment of their left knee to delay surgery because the pain in the affected joint is only mild. However, the compensatory gait mechanics that the patient may develop in an effort to reduce the pain associated with the affected joint may, as discussed above, have a lasting negative impact on the health of the patient's other joints (e.g., contralateral hip and lower back).

Thus, a system is needed that can monitor and analyze the potential effects of osteoarthritis on adjacent joints in order to treat the patient's holistic joint health. Referring back to the earlier example, due to the potential negative impacts on the adj acent joints, immediate knee replacement may be favored. US2013/0185310A1 relates to a method and system for human joint treatment plan and personalized surgery planning using 3D kinematics, fusion imaging and simulation. US2011/0112808A1 relates to integrated-model musculoskeletal therapies.

### SUMMARY

The invention is set out in the appended set of claims.

### BRIEF DESCRIPTION OF THE DRAWINGS

The accompanying drawings, which are incorporated in and form a part of the specification, illustrate the embodiments of the present disclosure and together with the written description serve to explain the principles, characteristics, and features of the present disclosure. In the drawings:
FIG. 1 depicts an illustration of an operating room with a patient monitoring system in accordance with certain embodiments as described herein.
FIG. 2 depicts a block diagram depicting a system in accordance with certain embodiments as described herein.
FIG. 3 depicts an illustrative data flow diagram depicting a method of determining outputs based on historical information according to an embodiment.
FIG. 4 depicts an illustrative example of a surgical navigation system according to an embodiment.
FIG. 5 illustrates a block diagram of an illustrative data processing system in which aspects of the illustrative embodiments are implemented.

### DETAILED DESCRIPTION

This disclosure is not limited to the particular systems, devices and methods described, as these may vary. The terminology used in the description is for the purpose of describing the particular versions or embodiments only and is not intended to limit the scope.

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention. As used in this document, the term "comprising" means "including, but not limited to."

Currently, some outcome scoring methods are used to attempt to standardize and evaluate the disease progression and associated symptoms of osteoarthritis. There are a number of validated scoring systems including, but not limited to, the Western Ontario and McMaster University Osteoarthritis Index (WOMAC), the Oxford-12 score (OKS), and the American Knee Society Scoring System (AKSS). Each of the knee scoring systems generally considers a number of factors including pain, stiffness, and physical function. While outcome scoring systems aim to provide a standardized measure of knee pain and function, their intended use is to merely gauge satisfaction levels after knee replacement surgery. Further, scoring systems like AKSS attempt to isolate pain and dysfunction at the joint of interest and purposefully neglect the systemic effects that osteoarthritis may have on the patient as a whole. Moreover, the results from outcome scoring systems are typically skewed due to the highly variable and subjective nature of what a "patient expectation" may be. Thus, it can be difficult to associate a successful operative outcome with a satisfied patient.

Accordingly, a system is needed which can assess the condition of a patient's knee, related to osteoarthritis, and quantify the systemic effects of the disease on both the affected joint as well as the patient's body as a whole. Stated differently, a system is needed that can provide a holistic estimation of the effects of forgoing surgery vs. undergoing surgery. Various embodiments are discussed herein that act as a guide for a "cost-benefit" style of analysis in order to determine whether a patient should undergo replacement surgery, not undergo, forgo, or delay joint replacement surgery.

Based on the foregoing, the present disclosure describes methods and systems to measure and quantitatively assess secondary effects (e.g., pain, joint dysfunction, cartilage damage, or muscle atrophy) caused by osteoarthritis. The various embodiments described herein relate to a system that performs joint reliability analysis using a forward-dynamic full-body simulation, which may then be used as a surgical planning and/or preventative treatment tool. Full body simulations may be seeded with patient specific data captured from one or more testing systems. As discussed herein, the patient-specific biomechanical modeling and simulation provides helpful insights for the loading environments within joints affected by osteoarthritis and the increased and/or irregular loading in adjacent joints. This quantitation of the systemic effects enables a statistical analysis and probabilistic forecasting in order to plan and create various treatment strategies.

In the following description, for purposes of explanation, numerous specific details are set forth in order to provide a thorough understanding of example embodiments. It will be evident to one skilled in the art, however, that embodiments can be practiced without these specific details.

In some embodiments, biomechanical data may be captured from a patient and used to construct a simulation model. As will be discussed further herein, the collection of a patient's biomechanical characterization may be accomplished via various measurement techniques including, but not limited to, gait analysis measurements with motion capture equipment and/or accelerometer data, fluoroscopy data, anthropometric measurements, radiological scans (e.g., computerized tomography scans or magnetic resonance imaging scans), strain gauge data, data from piezoelectric devices, force transducers, position sensors, infrared sensors or tracking devices, magnetic field data, signal triangulation data, radiofrequency identification tags/smart labels, Biodex balance characterization, electromyography muscle activation, radio wave data, data derived from computer-assisted surgical devices, radiosteriometric analysis, and the like.

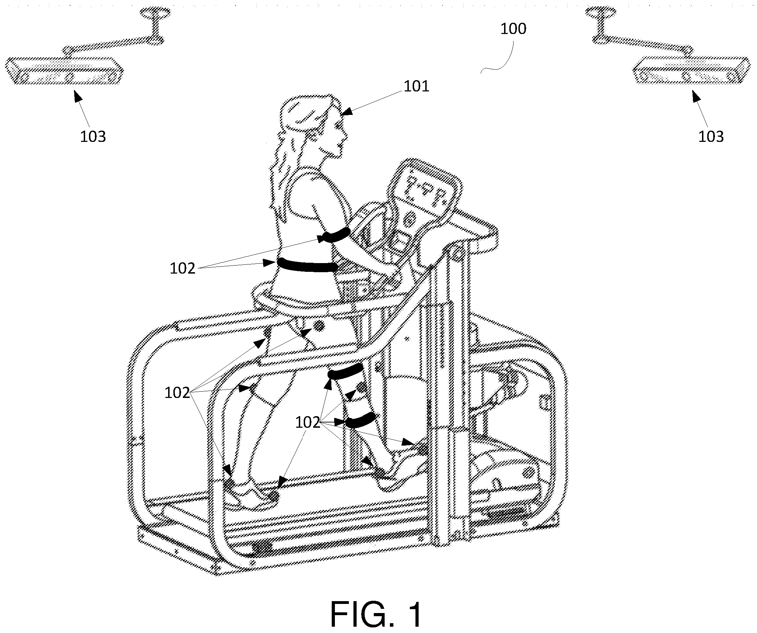

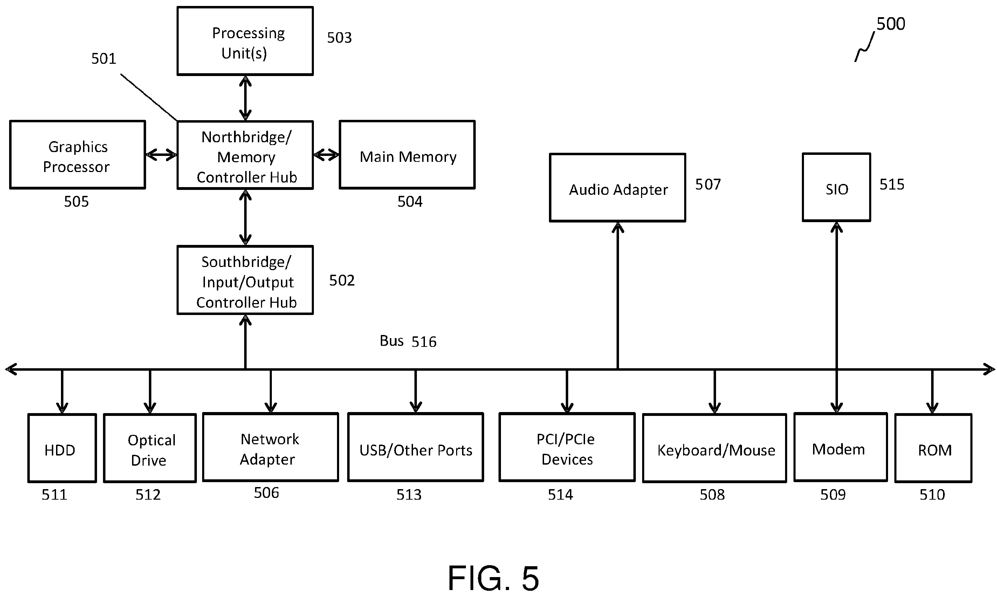

As noted above, the systems and methods described herein utilize biomechanical data captured from a patient to quantitatively assess secondary effects caused by osteoarthritis or other conditions that can affect patients' biomechanics. An example patient monitoring system 100 for capturing such biomechanical data is shown in FIG. 1. In one embodiment, the patient monitoring system 100 may be configured to capture and record a patient's 101 movement during various activities, such as walking on a treadmill as shown, via one or more tracking devices. The tracking devices can be configured to record various kinematic and/or anthropometric data from the patient. The tracking devices can include sensors 102 physically worn by or otherwise associated with the patient for sensing patient movement parameters and/or cameras 103, for example. The sensors 102 may be attached to the patient 101 at various points in order to record a patient's joint movement as they perform one or more activities. The sensors 102 can include accelerometers and/or piezoelectric devices, for example. The cameras 103 can include infrared cameras, for example. Further, the cameras 103 can be configured to track the movement of the patient and/or markers (e.g., passive or active markers) associated with the patient in order to record the patient's movement. In some embodiments, the sensors 102, cameras 103, and/or other tracking devices can be configured to transmit (e.g., via a wired or wireless connection) recorded patient information to a centralized processing device, such as the data processing system 500 described in connection with FIG. 5.

It should be understood that FIG. 1 is a non-limiting exemplary embodiment, and, as discussed herein, the activities performed by the patient (e.g., walking on a treadmill) may vary based on any number of factors, such as the patient's age, activity level, job requirement(s), hobbies, patient mobility preferences, and/or the like. Thus, in some embodiments, a patient may be required to perform various activities, such as, for example, normal walking, chair rise, chair sit, stair climb, stair descent, static standing, squat, jog, kneel, deep knee bend, turn gait, incline walking, decline walking, running, squatting, jumping, lifting objects, and/or even performing complex actions like swinging a golf club or baseball bat.

In some embodiments, a musculoskeletal simulation may require kinematic data and anthropometric data in order to estimate one or more forces within the joint. In addition to kinematic and anthropometric data, various embodiments may further utilize other data in generating or refining the biomechanical simulation. For example, one embodiment could use radiological scans (e.g., computerized tomography scans or magnetic resonance imaging scans) in order to provide a more exact anatomical model for the biomechanical simulation. Additionally or alternatively, an embodiment may utilize external force data (e.g., muscle activation or ground reaction forces) to further increase the confidence of the simulated results.

In some embodiments, biomechanical characterization may be conducted by subjecting the patient 101 to various activities involving the affected joint. As discussed herein, the activities may require varying levels of muscle input, angle of flexion/extension, and angle of internal/external joint rotation. A clinician may even tailor the activities performed to meet a patient's postoperative expectations. For example, active patients may choose more vigorous movements (e.g., jogging/running, kneeling, or performing a golf swing) while sedentary patients may do fewer or less rigorous movements (e.g., walking, performing a chair rise, or performing a knee bend).

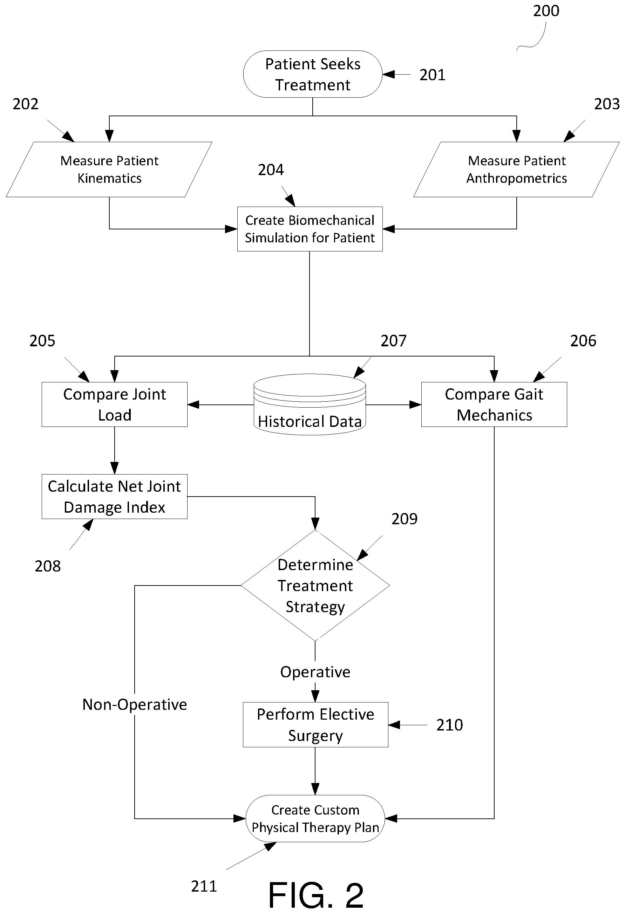

Patient monitoring systems, including the patient monitoring system 100 shown in FIG. 1, can be used in conjunction with various methods in order to construct a biomechanical simulation of a patient and determine an appropriate treatment strategy for the patient based on an analysis of the biomechanical simulation. A flow diagram 200 of one such method is shown in FIG. 2. Various portions or steps of the illustrated method can be performed by a computer system, such as the data processing system 500 shown in FIG. 5. Accordingly, various portions or steps of the illustrated method can be embodied as computer-executable instructions stored in a memory of the computer system that, when executed by a processor of the computer system, cause the computer system to perform the method.

In one embodiment, a patient may seek treatment 201 for osteoarthritis or another condition that affects the patient's movement. As discussed herein, the patient then performs various activities while the patient monitoring system 100 tracks their movement and/or monitors various physical characteristics of their body using the sensor(s) 102, camera(s) 103, and/or other tracking devices. Accordingly, an embodiment may capture/measure kinematic data 202 and anthropometric data 203 related to the specific patient.

A computer system including or coupled to the patient monitoring system 100 can receive the patient data, which can include the captured kinematic data 202 and/or anthropometric data 203, from the patient monitoring system. As discussed herein, the computer system can create 204 a biomechanical simulation specific to the patient based on the received patient data. Specifically, in some embodiments, physiological and biomechanical measurements (e.g., the kinematic data 202 and/or anthropometric data 203) may be used to create 204 a patient-specific biomechanical simulation that emulates the patient's musculoskeletal system during various activities. As would be understood by a person of ordinary skill in the art, there are a number of computational tools that may be used to create the simulation including, but not limited to, LifeModeler by LifeModeler, Inc., AnyBody Modeling System by AnyBody Technology, and OpenSimulator, which is an open source multi-platform. The patient-specific biomechanical simulation can be used or executed by the computer system to generate a variety of different biomechanical data related to the patient. The biomechanical data can be utilized to provide a holistic, whole-body assessment of the patient's biomechanical profile, which can in turn be used to assess whether the patient requires any therapy and, if so, which therapy or therapies would be most efficacious for the given patient. The biomechanical data can include, for example, a patient-specific joint loading profile and/or gait mechanics associated with various activities.

In some embodiments, the computer system can compare 205, 206 the joint load data and/or gait mechanics data to historical data 207 (i.e., historical joint load data and historical gait mechanics data) stored in a database (e.g., the database 440) coupled to or otherwise associated with the computer system. In some embodiments, the historical data 207 may comprise patient data that has been previously recorded using the methods and systems described herein. In a further embodiment, the historical data 207 may comprise data captured from healthy patient populations and/or historical data taken from one or more clinical studies. Thus, in some embodiments, the joint loading profile and associated biomechanical data may be compared 205, 206 to data from a population of healthy individuals to identify irregular mechanics and loading.

In a further embodiment, the computer system can calculate 208 a net joint damage index after the joint load has been compared 205 to the historical data 207. In some embodiments, the net joint damage index is a holistic metric that identifies the potential damage to any and all joints in the patient. In other words, the computer system can compare 205, 206 data associated with or generated from the patient specific biomechanical simulation to a large repository of historical data 207 to identify irregularities in the patient's movement. The identified irregularities can be evaluated by the computer system to determine the severity of those irregularities and/or calculate the potential negative effects (e.g., calculate 208 a net joint damage index) that such irregularities may have on the patient's other joints.

In another embodiment, the computer system can determine 209 a treatment strategy based on the calculated net joint damage index 208. In some embodiments, such as in the embodiment shown in FIG. 2, the determined 209 treatment strategy may include an operative solution (e.g., a recommendation to perform an elective surgery 210). Further, the computer system can create 211 a postoperative custom physical therapy plan to ensure the best possible postoperative results. Additionally or alternatively, the computer system can determine 209 a treatment strategy that is wholly non-operative (e.g., by creating 211 a custom physical therapy plan without recommending an associated surgical intervention).

In a non-limiting case study example, a patient may arrive at a clinic exhibiting joint pain (e.g., knee pain). As discussed herein, the clinician may outfit the patient with a number of sensors (e.g., the sensors 102 shown in FIG. 1) or markers for tracking the movement of the patient (e.g., via the cameras 103 shown in FIG. 1). For example, the patient may have one or more infrared (IR) markers attached for motion capture analysis. Additionally or alternatively, the patient may have one or more electromyography (EMG) electrodes attached to measure muscle firing patterns and intensities. A clinician may also record the patient's height, weight, distance between joint centers, and/or other anthropometric data. The clinician may sample the patient's kinematics, muscle activity, and ground reaction forces for squatting, walking on a treadmill, jogging on a treadmill, and/or the like. This recorded kinematic data and/or anthropometric data can be either directly received by a computer system or formatted and input into one or more software programs executed by a computer that leverages a musculoskeletal simulation engine, such as those discussed herein.

Continuing the non-limiting case study example, the force magnitudes in the patient's ankles, knees, and hips can be estimated from the simulation created by the one or more software programs executed by the computer system. Based on the quality and breadth of the input data, a confidence measurement may be provided along with the joint loading data. In addition, a comparison to normal joint loading patterns (e.g., as determined by examination of healthy populations or values taken from various prior clinical studies) is provided to identify the risk of overloading one or more of the patient's joint tissues. A side-by-side comparison of the patient's simulated biomechanics and the biomechanics of a healthy individual are presented to the clinician to aid in identification of irregular or compensatory mechanisms.

In one embodiment, the joint loading data may be used in conjunction with data taken from one or more *in vitro* cartilage wear studies in order to estimate the longevity of the patient's joint tissues in the current loading scheme. This metric may be used by the patient and clinician as a decision aid when developing a treatment strategy. In a further embodiment, the patient specific simulation may be altered to predict the effects of various treatments including both surgical and non-invasive treatments. For example, the effect of physical therapy could be estimated by altering the influence of various muscle groups and/or the impact of orthotics on joint loading could be simulated. The results from the simulation can be summarized in a report that characterizes the patient's holistic joint health. The report can be generated by the one or more software programs executed by the computer system and provided to the clinician.

In the above described example case study, the data input and modelling may be automated, which can require a robust simulation platform and a consistent data format. However, in some embodiments, a service model may be used instead. In this embodiment, the clinician may be responsible for collecting patient data and sending it to an analysis center. Upon receipt of the data, a modelling and simulation expert can compile and tune the patient-specific simulation in order to generate joint loading results and other biomechanical data utilizing a biomechanical simulation, as described above.

Either alone or in cooperation with the clinician, the modelling expert can perform virtual procedures on the patient using the biomechanical simulation to estimate the effects on the patient's joint loading and other biomechanics. For example, the modelling expert could employ one or more 3D models of a joint replacement prosthesis positioned according to the clinician's instructions and/or feedback. The impact of the joint replacement surgery on the loading scheme of the other joints could then be estimated. Similarly, the loading of the affected and unaffected joints, in tandem with data from healthy populations or wear data from *in vitro* cartilage studies, can be used to assess the holistic joint health of the patient and guide treatment strategy.

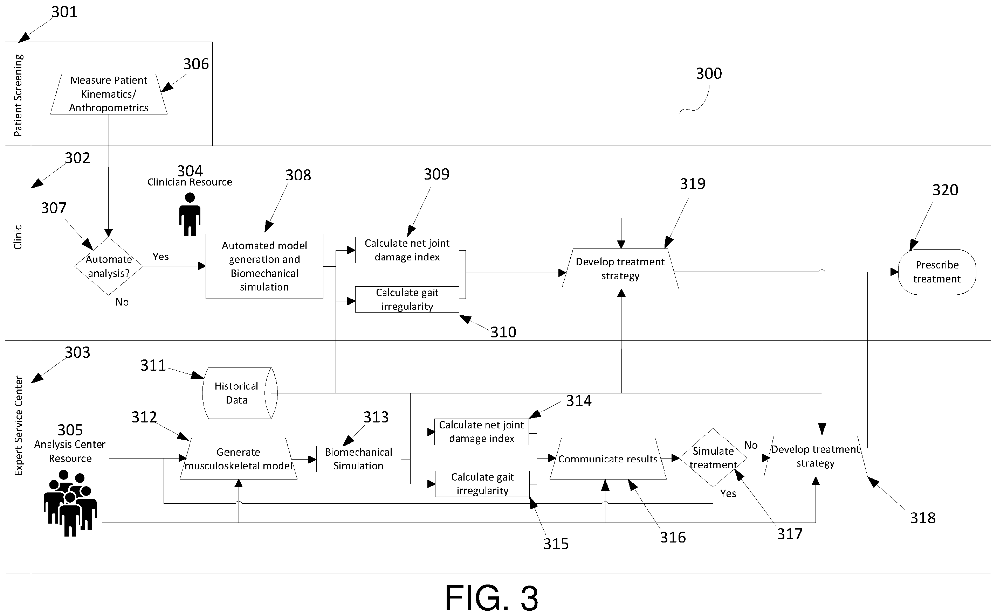

FIG. 3 illustrates a block diagram 300 demonstrating how the various systems, components or method steps described herein could be divided among different groups of individuals or organizations based on how and when each step/component of the system is performed. It should be appreciated that FIG. 3 is for illustrative purposes only and that various steps/components may be performed at different times, in different locations, and by various individuals (e.g., a clinician 304 or an analysis center 305). Thus, according to some embodiments, the measurement of the kinematics and/or anthropometrics 306 may be carried out in a screening process 301. In a further embodiment, the automated analysis 307 and the creation of a biomechanical simulation 308 may be performed in a clinic setting 302. As discussed herein, the biomechanical simulation 308 may be used to calculate the net joint damage 309 and the gait irregularity 310.

In one embodiment, the calculation of the net joint damage 309 and gait irregularity 310 can be based upon a comparison between the generated biomechanical simulation and historical data 311. In a further embodiment, the expert service center 303 may execute various steps, such as, generating a musculoskeletal model 312 and creating a biomechanical simulation 313 (which can be performed in addition to or in lieu of the creation of the biomechanical simulation 308 in the clinic 302). As shown, the generation of a musculoskeletal model 312 may consider input from both the clinic 302 and an analysis center resource 305 (e.g., one or more modeling experts). Similar to the clinic 302, the expert service center 303 may also calculate a net joint damage index 314 and gait irregularity 315. The results may be communicated 316 to another party (e.g., to the clinician 304 and/or analysis center 305) to determine whether treatment should be simulated 317. If a treatment simulation is requested, an embodiment may generate a new musculoskeletal model 312 based on an identified treatment plan.

Accordingly, the musculoskeletal model 312 can be passed through the biomechanical simulation 313 again and a new net j oint damage index 314 and gait irregularity 315 can be calculated. This process can be repeated until an appropriate treatment has been identified. Once an approved treatment has been determined (e.g., no further simulation is required), the service center 303 and/or clinic 302 can develop 318, 319 a treatment strategy. In one embodiment, the service center 303 and/or clinic 302 can determine a final treatment based on one or more of the developed 318, 319 treatment strategies and the final treatment can then be prescribed 320 to the patient.

It should be understood that although the majority of the disclosure relates to osteoarthritis of the knee, similar techniques may be used to quantify the systemic effects of osteoarthritis in other joints (e.g., the hip and/or the ankle). Furthermore, the technique disclosed herein is not specific to osteoarthritis. Any conditions that affect biomechanics, such as congenital skeletal deformities and/or sports injuries to the soft tissues, may also be analyzed to determine the best treatment method.

As discussed, a treatment simulation 317 for the patient may be extended to include simulations of various treatment options. In one embodiment, a clinician may utilize the biomechanical simulation 308 and/or 313 to conduct virtual interventions and provide insight on the effectiveness of various treatments. For example, the clinician may conduct a virtual joint replacement surgery on the affected joint by altering the model of the effected bones (i.e., resection) and replacing the joint with 3D models of knee replacement implants.

In a further embodiment, the clinician may further use the simulation tool to optimize surgical parameters, such as the make, size, and position of an implant and/or implant components, which can then be altered and optimized to elicit a desired biomechanical response. In some embodiments, this optimization may be aimed at normalizing joint loads to "balance" the biomechanical system and minimize joint deterioration over time. Additionally or alternatively, an embodiment may optimize one or more joint replacement surgery parameters and output such parameters to a robotic surgical system or computer-aided surgical system, thereby providing an optimal implant location and position based on the simulation's determined optimal orientation.

In a further embodiment, the optimized joint replacement parameters may be realized through the use of a 3D printed patient-specific instrumentation (e.g., Smith and Nephew VISIONAIRE cutting guides). VISIONAIRE is a registered trademark of Smith & Nephew, Inc. The simulation may be used to characterize the effect of any non-surgical treatment strategies (e.g., bracing the joint, using custom orthotics, or performing physical therapy). In another embodiment, the simulation tool may be used to characterize foot mechanics and the effect of the customized orthotic correction. Thus, orthotic design may be optimized to restore proper knee function and the resulting design may be fabricated using any known means of manufacture, including additive manufacturing (i.e., 3D printing).

In another embodiment, one or more software packages may be used to predict a likely disease progression trajectory. This, as discussed herein, can be accomplished by capturing the patient's biomechanical data at discrete time points (e.g., using the patient monitoring system 100 shown in FIG. 1). Data capture scheduling can be tailored to fit the patient's needs and testing may be administered at regular or irregular intervals (e.g., yearly, monthly, or bi-annually). A comparison of the simulation-derived joint loading profile at these time points may then be used to identify trends or changes in the irregular loading and biomechanics. In one embodiment, these loading trends may be extrapolated to suggest the systemic effect of the joint disease at a future date. As discussed herein, this knowledge may be used in a cost-benefit style of analysis to determine whether a patient should immediately pursue surgery or surgery could/should be deferred until a later date. Stated differently, if the likelihood of damage to adjacent joints (i.e., the cost) is greater than the risk associated with early intervention (i.e., the benefit), the clinician may suggest that the patient immediately schedule a partial or total knee replacement.

In another embodiment, simulation software may be used in conjunction with a database of patient reported outcome measures (PROMS). In this embodiment, the following information may be captured for each patient in the database: biomechanical data, simulation data, whole body joint loading profile, treatment history, and outcome measures. Through the use of machine learning and other known data analysis techniques, the software can be programmed to identify (i) other patients that have similar biomechanics and/or joint loading to the patient being analyzed and (ii) a treatment strategy that is likely to produce favorable outcomes for the patient.

In some embodiments, the system may be used in combination with a robotic surgical system, such as Smith & Nephew's NAVIO surgical system or another computer-assisted surgical (CAS) system to post-operatively assess the success of the surgery. NAVIO is a registered trademark of Blue Belt Technologies, Inc., which is a subsidiary of Smith & Nephew, Inc. Most robotic and computer-assisted surgical systems capture bone anatomy and the size and relative location of implant components. This positional data may be used to inform a simulation tool (e.g., LifeModeler, AnyBody, OpenSim, or LifeMOD/KneeSim) of a patient-specific simulation that demonstrates the expected loading based on the implant components. The implant loading information, which is generated by the simulation, along with engineering data and clinical performance data, may be used to estimate the longevity of the implant components in their expected loading scheme. Therefore, in some embodiments, the procedure may be scored and the likelihood of revision surgery can be accurately estimated. Furthermore, the postoperative loading scheme may be linked to the database of patient reported outcome measures (PROMS) from previous patients in order to predict the long-term success of the procedure.

In some embodiments, the systems and methods described herein can further be incorporated into systems and methods for generating and updating intraoperative surgical plans, such as are described in PCT Application No. PCT/US20/16612, titled COMPUTER-ASSISTED ARTHROPLASTY SYSTEM TO IMPROVE PATELLAR PERFORMANCE, filed February 4, 2020. For example, a computer system executing the method(s) shown in FIGS. 2 and/or 3 can use (i) the kinematic data (e.g., as detected via a patient monitoring system 100) and/or anthropometric data received by the computer system or (ii) data derived from the biomechanical simulation generated by the computer system (e.g., joint loading data or gait mechanics data) to determine or update an implant size, type, location, or orientation or other intraoperative parameters associated with a surgical plan. As a further example, a computer system could determine a severity of a patient' s joint damage according to the joint loading data and/or gait mechanics data derived from the biomechanical simulation and then updated the implant size, type, location, or orientation accordingly. Additionally or alternatively, the systems and methods described herein could be incorporated into an operative patient care system, as is also described in PCT Application No. PCT/US20/16612. For example, a computer system executing the method(s) shown in FIGS. 2 and/or 3 could use the kinematic data, anthropometric data, and/or data derived from the biomechanical simulation to develop a detailed surgical case plan with distinct steps that are monitored and/or executed using a computer-aided surgical system.

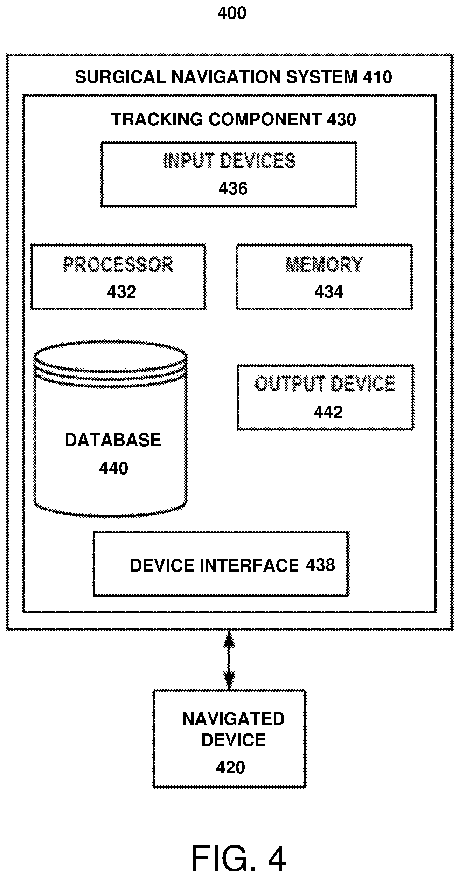

FIG. 4 illustrates components of a surgical navigation system 400 that can be configured to perform knee movement tracking according to some embodiments. The surgical navigation system 400 can assist a surgeon in performing certain surgical procedures, such as knee replacement revision surgery, but can also be used for procedures involving other joints.

Referring to FIG. 4, an exemplary surgical system, generally designated as 400, is shown. The exemplary surgical system 400 can be configured to intraoperatively obtain positional data relating to a range of motion of a knee that will be subject to a surgical procedure. This positional data can correspond to discrete angles of flexion or extension of the operative knee. In some examples, additional information such as soft tissue and anatomical structure information can be intraoperatively collected as well. In some examples, ligament attachment locations can be intraoperatively and/or pre-operatively collected as well.

In certain embodiments, the system 400 can include a surgical navigation system 410 and a navigated device 420, such as a point probe, a cutting device, or another surgical tool that may be used during a surgical procedure. In operation, the knee of a patient is moved through a range of motion, so that the position of various anatomical components, such as the femur, the tibia, and the patella, can be tracked. Additional information such as soft tissue information including ligament strain can also be intraoperatively collected as described herein.

In certain implementations, the surgical navigation system 410 can be configured to employ a tracking component 430. The tracking component 430 can be configured and implemented as an integral system or component within the surgical navigation system 410 or as a standalone component that connects to the surgical navigation system. It is to be appreciated that embodiments of the described subject matter can be implemented by various types of operating environments, computer networks, platforms, frameworks, computer architectures, and/or computing devices.

In some embodiments, the surgical navigation system 410 and/or the tracking component 430 may include one or more processors 432 and memory devices 434, as well as various input/output devices 436, communication interfaces 438 (e.g., for facilitating communications between the navigated device 420 and the tracking component 430), and/or other types of devices. The surgical navigation system 410 and/or the tracking component 430 can include a combination of hardware and software.

In additional embodiments, the surgical navigation system 410 and/or the tracking component 430 may implement and utilize one or more program modules. Generally, program modules include routines, programs, objects, components, data structures, and/or the like that perform particular tasks or implement particular abstract data types. In a further embodiment, the surgical navigation system 410 and/or the tracking component 430 can be implemented by one or more computing devices configured to provide various types of services and/or data stores in accordance with aspects of the described subject matter. Exemplary computing devices can include, without limitation: personal computing devices, web servers, front end servers, application servers, database servers, domain controllers, domain name servers, directory servers, and/or other suitable computers. Components of the surgical navigation system 410 and/or the tracking component 430 can be implemented by software, hardware, firmware or a combination thereof.

The tracking component 430 can include a processor 432, memory 434, input devices 436, a communication interface 438, a measurement database 440, and an output device 442. The input devices 436 can be configured and implemented to receive instructions from a surgeon before implementing a surgical plan.

The tracking component 430 can be configured to receive and characterize various anatomical information related to the knee undergoing surgery. For example, the processor 432 can be configured to execute software instructions stored in memory 434 to determine surface information for the tibia, femur, and/or patella from the navigated device 420 before the tracking component 430 evaluates movement information. In some embodiments, relative positions and specific information, such as ligament strain and/or laxity, can be determined by the processor 432 based upon information received from a navigated device, such as a force or strain gauge, or by measuring movement and varus/valgus information as the knee is moved through its full range of motion. The output device 442 can generate position measurements of various anatomical components and/or display portions of the surgical plan as described herein.

In another embodiment, an operative patient care system may be used to optimize outcomes and patient satisfaction for total joint arthroplasty. The system may be designed to use patient characteristic data, along with clinician data, healthcare facility data, and historical outcome data to develop an algorithm that suggests an optimal treatment (preoperative, operative, and postoperative) plan based on a desired clinical outcome, such as that described in FIG. 2. The system may track adherence to the suggested plan and may adapt the plan based on the performance of the patient, surgeon, or care provider. Once the surgical treatment plan for a particular patient is complete, collected data may be logged in a historical database (e.g., historical data 207) that is accessible for future patients and the development of future treatment plans.

In some embodiments, a data collecting and management system may provide a detailed surgical case plan with distinct steps that are monitored and/or executed using a computer-assisted surgical system. The performance of one or more users may be determined at the completion of each step in the surgical treatment plan and may be used to suggest changes to subsequent steps.

Referring now to FIG. 5, a block diagram of an illustrative data processing system 500 is shown, within which some aspects of the illustrative embodiments discussed herein are implemented. The data processing system 500 is an example of a computer, such as a server or client, in which computer usable code or instructions implementing the process for illustrative embodiments of the present invention are located. In some embodiments, the data processing system 500 may be a server computing device.

In the depicted example, data processing system 500 can employ a hub architecture including a northbridge and memory controller hub (NB/MCH) 501 and southbridge and input/output (I/O) controller hub (SB/ICH) 502. Processing unit 503, main memory 504, and graphics processor 505 can be connected to the NB/MCH 501. Graphics processor 505 can be connected to the NB/MCH 501 through, for example, an accelerated graphics port (AGP).

In the depicted example, a network adapter 506 connects to the SB/ICH 502. An audio adapter 507, keyboard and mouse adapter 508, modem 509, read only memory (ROM) 510, hard disk drive (HDD) 511, optical drive (e.g., CD or DVD) 512, universal serial bus (USB) ports and other communication ports 513, and PCI/PCIe devices 514 may connect to the SB/ICH 502 through bus system 516. PCI/PCIe devices 514 may include Ethernet adapters, add-in cards, and PC cards for notebook computers. ROM 510 may be, for example, a flash basic input/output system (BIOS). The HDD 511 and optical drive 512 can use an integrated drive electronics (IDE) or serial advanced technology attachment (SATA) interface. A super I/O (SIO) device 515 can be connected to the SB/ICH 502.

An operating system can run on the processing unit 503. The operating system can coordinate and provide control of various components within the data processing system 500. As a client, the operating system can be a commercially available operating system. An object-oriented programming system, such as the Java^{®} programming system, may run in conjunction with the operating system and provide calls to the operating system from the object-oriented programs or applications executing on the data processing system 500. As a server, the data processing system 500 can be an IBM^{®} eServer System p^{®} running the Advanced Interactive Executive operating system or the Linux operating system. The data processing system 500 can be a symmetric multiprocessor (SMP) system that can include a plurality of processors in the processing unit 503. Alternatively, a single processor system may be employed.

Instructions for the operating system, the object-oriented programming system, and applications or programs are located on storage devices, such as the HDD 511, and are loaded into the main memory 504 for execution by the processing unit 503. The processes for embodiments described herein can be performed by the processing unit 503 using computer usable program code, which can be located in a memory such as, for example, main memory 504, ROM 510, or in one or more peripheral devices.

A bus system 516 can be comprised of one or more busses. The bus system 516 can be implemented using any type of communication fabric or architecture that can provide for a transfer of data between different components or devices attached to the fabric or architecture. A communication unit such as the modem 509 or the network adapter 506 can include one or more devices that can be used to transmit and receive data.

Those of ordinary skill in the art will appreciate that the hardware depicted in FIG. 5 may vary depending on the implementation. Other internal hardware or peripheral devices, such as flash memory, equivalent non-volatile memory, or optical disk drives may be used in addition to or in place of the hardware depicted. Moreover, the data processing system 500 can take the form of any of a number of different data processing systems, including but not limited to, client computing devices, server computing devices, tablet computers, laptop computers, telephone or other communication devices, personal digital assistants, and the like. Essentially, data processing system 500 can be any known or later developed data processing system without architectural limitation.

While various illustrative embodiments incorporating the principles of the present teachings have been disclosed, the present teachings are not limited to the disclosed embodiments. Instead, this application is intended to cover any variations, uses, or adaptations of the present teachings and use its general principles. Further, this application is intended to cover such departures from the present disclosure that are within known or customary practice in the art to which these teachings pertain.

In the above detailed description, reference is made to the accompanying drawings, which form a part hereof. In the drawings, similar symbols typically identify similar components, unless context dictates otherwise. The illustrative embodiments described in the present disclosure are not meant to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein are generally intended as "open" terms (for example, the term "including" should be interpreted as "including but not limited to," the term "having" should be interpreted as "having at least," the term "includes" should be interpreted as "includes but is not limited to," et cetera). While various compositions, methods, and devices are described in terms of "comprising" various components or steps (interpreted as meaning "including, but not limited to"), the compositions, methods, and devices can also "consist essentially of" or "consist of" the various components and steps, and such terminology should be interpreted as defining essentially closed-member groups.

In addition, even if a specific number is explicitly recited, those skilled in the art will recognize that such recitation should be interpreted to mean at least the recited number (for example, the bare recitation of "two recitations," without other modifiers, means at least two recitations, or two or more recitations). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, "a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, sample embodiments, or drawings, should be understood to contemplate the possibilities of including one of the terms, either of the terms, or both terms. For example, the phrase "A or B" will be understood to include the possibilities of "A" or "B" or "A and B."

In addition, where features of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera. As a non-limiting example, each range discussed herein can be readily broken down into a lower third, middle third and upper third, et cetera. As will also be understood by one skilled in the art all language such as "up to," "at least," and the like include the number recited and refer to ranges that can be subsequently broken down into subranges as discussed above. Finally, as will be understood by one skilled in the art, a range includes each individual member. Thus, for example, a group having 1-3 components refers to groups having 1, 2, or 3 components. Similarly, a group having 1-5 components refers to groups having 1, 2, 3, 4, or 5 components, and so forth.

The term "about," as used herein, refers to variations in a numerical quantity that can occur, for example, through measuring or handling procedures in the real world; through inadvertent error in these procedures; through differences in the manufacture, source, or purity of compositions or reagents; and the like. Typically, the term "about" as used herein means greater or lesser than the value or range of values stated by 1/10 of the stated values, e.g., ±10%. The term "about" also refers to variations that would be recognized by one skilled in the art as being equivalent so long as such variations do not encompass known values practiced by the prior art. Each value or range of values preceded by the term "about" is also intended to encompass the embodiment of the stated absolute value or range of values. Whether or not modified by the term "about," quantitative values recited in the present disclosure include equivalents to the recited values, e.g., variations in the numerical quantity of such values that can occur, but would be recognized to be equivalents by a person skilled in the art.

## Claims

1. A computer-implemented method comprising:
receiving, by a computer system, patient data comprising kinematic data (202) and anthropometric data (203);
creating (204), by the computer system, a patient-specific biomechanical simulation (308, 313) based on the patient data;
calculating (208), by the computer system, at least one of joint loading data or gait mechanics data based on the patient-specific biomechanical simulation (308, 313);
comparing (205, 206), by the computer system, at least one of the joint loading data or the gait mechanics data to historical data (207); and
determining (209), by the computer system, a treatment strategy based on the comparison;
further comprising calculating, by the computer system, a net joint damage index based on the joint loading data compared to the historical data, wherein the net joint damage index comprises a net measure of potential joint damage suffered by a patient (101), and wherein the determined treatment strategy is further based on the net joint damage index,
**characterised in that** the historical data comprises a plurality of previously created biomechanical simulations (308, 313) of patients (101).

2. The computer-implemented method of claim 1, wherein the patient data received from a patient monitoring system.

3. The computer-implemented method of any of claims 1-2, further comprising:
simulating, by the computer system, a plurality of different treatment strategies using the patient-specific biomechanical simulation (308, 313), wherein the determined treatment strategy is determined from the plurality of simulated different treatment strategies.

4. The computer-implemented method of claim 1, further comprising:
transmitting, by the computer system, the patient-specific biomechanical simulation (308, 313) to at least one of a computer-assisted surgical system or a clinician computer system.

5. The computer-implemented method of any of claims 1-4, wherein the treatment strategy comprises a non-operative intervention.

6. The computer-implemented method of claim 5, wherein the non-operative intervention comprises a recommendation for at least one of physical therapy of using a brace.

7. The computer-implemented method of any of claims 1-4, wherein the treatment strategy comprises an operative intervention.

8. The computer-implemented method of claim 7, wherein the operative intervention comprises a total or partial joint replacement surgery.

9. A computer system comprising:
a processor; and
a memory coupled to the processor, the memory storing instructions that, when executed by the processor, cause the computer system to:
receive patient data comprising kinematic data (202) and anthropometric data (203);
create (205) a patient-specific biomechanical simulation (308, 313) based on the patient data;
calculate (208) at least one of joint loading data or gait mechanics data based on the patient-specific biomechanical simulation (308, 313);
compare (205, 206) at least one of the joint loading data or the gait mechanics data to historical data (207); and
determine (209) a treatment strategy based on the comparison,
wherein the memory further stores instructions that, when executed by the processor, cause the computer system to calculate a net joint damage index based on the joint loading data compared to the historical data, wherein the net joint damage index comprises a net measure of potential joint damage suffered by a patient (101), and wherein the determined treatment strategy is further based on the net joint damage index,
**characterised in that** the historical data comprises a plurality of previously created biomechanical simulations (308, 313) of patients (101).

10. The computer system of claim 9, wherein the patient data is at least partially sensed via a patient monitoring system to which the computer system is communicably coupled.

11. The computer system of any of claims 9-10, wherein the memory further stores instructions that, when executed by the processor, cause the computer system to:
simulate a plurality of different treatment strategies using the patient-specific biomechanical simulation (308, 313), wherein the determined treatment strategy is further based on the plurality of simulated different treatment strategies.

## Patentansprüche

1. Computerimplementiertes Verfahren, umfassend:
Empfangen, durch ein Computersystem, von Patientendaten, die kinematische Daten (202) und anthropometrische Daten (203) umfassen;
Erstellen (204), durch das Computersystem, einer patientenspezifischen biomechanischen Simulation (308, 313) basierend auf den Patientendaten;
Berechnen (208), durch das Computersystem, wenigstens eines von Gelenkbelastungsdaten oder Gangmechanikdaten basierend auf der patientenspezifischen biomechanischen Simulation (308, 313);
Vergleichen (205, 206), durch das Computersystem, wenigstens eines von den Gelenkbelastungsdaten oder den Gangmechanikdaten mit historischen Daten (207); und
Bestimmen (209), durch das Computersystem, einer Behandlungsstrategie basierend auf dem Vergleich;
ferner umfassend das Berechnen, durch das Computersystem, eines Netto-Gelenkschadenindexes basierend auf den Gelenkbelastungsdaten im Vergleich zu den historischen Daten, wobei der Netto-Gelenkschadenindex ein Nettomaß potenzieller Gelenkschäden umfasst, die ein Patient (101) erlitten hat, und wobei die bestimmte Behandlungsstrategie ferner auf dem Netto-Gelenkschadenindex basiert, **dadurch gekennzeichnet, dass** die historischen Daten mehrere zuvor erstellte biomechanische Simulationen (308, 313) von Patienten (101) umfassen.

2. Computerimplementiertes Verfahren nach Anspruch 1, wobei die Patientendaten von einem Patientenüberwachungssystem empfangen werden.

3. Computerimplementiertes Verfahren nach einem der Ansprüche 1-2, ferner umfassend:
Simulieren, durch das Computersystem, mehrerer unterschiedlicher Behandlungsstrategien unter Verwendung der patientenspezifischen biomechanischen Simulation (308, 313), wobei die bestimmte Behandlungsstrategie anhand der mehreren simulierten unterschiedlichen Behandlungsstrategien bestimmt wird.

4. Computerimplementiertes Verfahren nach Anspruch 1, ferner umfassend:
Übertragen, durch das Computersystem, der patientenspezifischen biomechanischen Simulation (308, 313) an wenigstens eines von einem computergestützten chirurgischen System oder einem Klinikercomputersystem.

5. Computerimplementiertes Verfahren nach einem der Ansprüche 1-4, wobei die Behandlungsstrategie einen nichtoperativen Eingriff umfasst.

6. Computerimplementiertes Verfahren nach Anspruch 5, wobei der nichtoperative Eingriff eine Empfehlung für wenigstens eines von Physiotherapie oder Verwenden einer Stütze umfasst.

7. Computerimplementiertes Verfahren nach einem der Ansprüche 1-4, wobei die Behandlungsstrategie einen operativen Eingriff umfasst.

8. Computerimplementiertes Verfahren nach Anspruch 7, wobei der operative Eingriff eine vollständige oder teilweise Gelenkersatzoperation umfasst.

9. Computersystem, umfassend:
einen Prozessor; und
einen Speicher, der an den Prozessor gekoppelt ist, wobei der Speicher Anweisungen speichert, die, wenn sie von dem Prozessor ausgeführt werden, das Computersystem hierzu veranlassen:
Empfangen von Patientendaten, die kinematische Daten (202) und anthropometrische Daten (203) umfassen;
Erstellen (205) einer patientenspezifischen biomechanischen Simulation (308, 313) basierend auf den Patientendaten;
Berechnen (208) wenigstens eines von Gelenkbelastungsdaten oder Gangmechanikdaten basierend auf der patientenspezifischen biomechanischen Simulation (308, 313);
Vergleichen (205, 206) wenigstens eines von Gelenkbelastungsdaten oder Gangmechanikdaten mit historischen Daten (207); und
Bestimmen (209) einer Behandlungsstrategie basierend auf dem Vergleich,
wobei der Speicher ferner Anweisungen speichert, die, wenn sie von dem Prozessor ausgeführt werden, das Computersystem veranlassen, einen Netto-Gelenkschadenindex basierend auf den Gelenkbelastungsdaten im Vergleich zu den historischen Daten zu berechnen, wobei der Netto-Gelenkschadenindex ein Nettomaß potenzieller Gelenkschäden umfasst, die ein Patient (101) erlitten hat, und wobei die bestimmte Behandlungsstrategie ferner auf dem Netto-Gelenkschadenindex basiert, **dadurch gekennzeichnet, dass** die historischen Daten mehrere zuvor erstellte biomechanische Simulationen (308, 313) von Patienten (101) umfassen.

10. Computersystem nach Anspruch 9, wobei die Patientendaten wenigstens teilweise über ein Patientenüberwachungssystem erfasst werden, an welches das Computersystem kommunikativ gekoppelt ist.

11. Computersystem nach einem der Ansprüche 9-10, wobei der Speicher ferner Anweisungen speichert, die, wenn sie von dem Prozessor ausgeführt werden, das Computersystem hierzu veranlassen:
Simulieren mehrerer unterschiedlicher Behandlungsstrategien unter Verwendung der patientenspezifischen biomechanischen Simulation (308, 313), wobei die bestimmte Behandlungsstrategie ferner auf den mehreren simulierten unterschiedlichen Behandlungsstrategien basiert.

## Revendications

1. Procédé mis en œuvre par ordinateur comprenant :
la réception, par un système informatique, de données patient comprenant des données cinématiques (202) et des données anthropométriques (203) ;
la création (204), par le système informatique, d'une simulation biomécanique spécifique au patient (308, 313) sur la base des données patient ;
le calcul (208), par le système informatique, d'au moins une des données de charge articulaire ou des données de mécanique de la marche sur la base de la simulation biomécanique spécifique au patient (308, 313) ;
la comparaison (205, 206), par le système informatique, d'au moins l'une des données de charge articulaire ou des données de mécanique de la marche à des données historiques (207) ; et
la détermination (209), par le système informatique, d'une stratégie de traitement sur la base de la comparaison ;
comprenant en outre le calcul, par le système informatique, d'un indice de lésion articulaire nette sur la base des données de charge articulaire comparées aux données historiques, l'indice de lésion articulaire nette comprenant une mesure nette de la lésion articulaire potentielle subie par un patient (101), et la stratégie de traitement déterminée étant en outre basée sur l'indice de lésion articulaire nette, **caractérisé en ce que** les données historiques comprennent une pluralité de simulations biomécaniques (308, 313) de patients (101) créées précédemment.

2. Procédé mis en œuvre par ordinateur selon la revendication 1, les données patient étant reçues d'un système de surveillance de patient.

3. Procédé mis en œuvre par ordinateur selon l'une quelconque des revendications 1 et 2, comprenant en outre :
la simulation, par le système informatique, d'une pluralité de stratégies de traitement différentes à l'aide de la simulation biomécanique spécifique au patient (308, 313), la stratégie de traitement déterminée étant déterminée à partir de la pluralité de stratégies de traitement différentes simulées.

4. Procédé mis en œuvre par ordinateur selon la revendication 1, comprenant en outre :
la transmission, par le système informatique, de la simulation biomécanique spécifique au patient (308, 313) à au moins l'un d'un système chirurgical assisté par ordinateur ou d'un système informatique clinique.

5. Procédé mis en œuvre par ordinateur selon l'une quelconque des revendications 1 à 4, la stratégie de traitement comprenant une intervention non chirurgicale.

6. Procédé mis en œuvre par ordinateur selon la revendication 5, l'intervention non chirurgicale comprenant une recommandation pour au moins l'un parmi une thérapie physique et l'utilisation d'une orthèse.

7. Procédé mis en œuvre par ordinateur selon l'une quelconque des revendications 1 à 4, la stratégie de traitement comprenant une intervention chirurgicale.

8. Procédé mis en œuvre par ordinateur selon la revendication 7, l'intervention chirurgicale comprenant une chirurgie de remplacement total ou partiel d'une articulation.

9. Système informatique comprenant :
un processeur ; et
une mémoire couplée au processeur, la mémoire stockant des instructions qui, lorsqu'elles sont exécutées par le processeur, amènent le système informatique à :
recevoir des données patient comprenant des données cinématiques (202) et des données anthropométriques (203) ;
créer (205) une simulation biomécanique spécifique au patient (308, 313) sur la base des données patient ;
calculer (208) au moins l'une des données de charge articulaire ou des données de mécanique de la marche sur la base de la simulation biomécanique spécifique au patient (308, 313) ;
comparer (205, 206) au moins l'une des données de charge articulaire ou des données de mécanique de la marche à des données historiques (207) ; et
déterminer (209) une stratégie de traitement sur la base de la comparaison,
la mémoire stockant en outre des instructions qui, lorsqu'elles sont exécutées par le processeur, amènent le système informatique à calculer un indice de lésion articulaire nette sur la base des données de charge articulaire comparées aux données historiques, l'indice de lésion articulaire nette comprenant une mesure nette de la lésion articulaire potentielle subie par un patient (101), et la stratégie de traitement déterminée étant en outre basée sur l'indice de lésion articulaire nette, **caractérisé en ce que** les données historiques comprennent une pluralité de simulations biomécaniques (308, 313) de patients (101) créées précédemment.

10. Système informatique selon la revendication 9, les données patient étant au moins partiellement détectées par l'intermédiaire d'un système de surveillance de patient auquel le système informatique est couplé de manière communicative.

11. Système informatique selon l'une quelconque des revendications 9 et 10, la mémoire stockant en outre des instructions qui, lorsqu'elles sont exécutées par le processeur, amènent le système informatique à :
simuler une pluralité de stratégies de traitement différentes à l'aide de la simulation biomécanique spécifique au patient (308, 313), la stratégie de traitement déterminée étant en outre basée sur la pluralité de stratégies de traitement différentes simulées.
